# EUROPEAN PATENT APPLICATION

(11) **EP 4 183 881 A1**
(43) Date of publication of application: **24.05.2023**
(21) Application number: 21847199.3
(22) Date of filing: 13.07.2021
(51) Int. Cl.: C12N 15/113, C12N 15/77, C12N 15/67, C12P 13/04, C12R 1/15

(54) **MUTANT OF PYRUVATE CARBOXYLASE GENE PROMOTER AND USE THEREOF**

(30) Priority: 20.07.2020 CN 202010696983
(71) Applicant: Tianjin Institute of Industrial Biotechnology, Chinese Academy of Sciences, Tianjin Airport Economic Area Tianjin 300308 (CN)
(72) Inventor: SUN, Jibin, Tianjin 300308 (CN); LIU, Jiao, Tianjin 300308 (CN); ZHENG, Ping, Tianjin 300308 (CN); SHI, Tuo, Tianjin 300308 (CN); ZHOU, Wenjuan, Tianjin 300308 (CN); CHEN, Jiuzhou, Tianjin 300308 (CN); GUO, Xuan, Tianjin 300308 (CN); MA, Yanhe, Tianjin 300308 (CN)
(74) Representative: Heubeck, Christian
(86) International application number: PCT/CN2021/105989
(87) International publication number: WO 2022/017223

(57) **Abstract**

Disclosed are a mutant of a pyruvate carboxylase gene promoter of *Corynebacterium glutamicum* and applications thereof. The mutant has improved promoter activity compared with a wild-type promoter, and can be used for enhancing expression of a target gene, for example, operably ligating the mutant to a pyruvate carboxylase gene enhances the expression intensity of the pyruvate carboxylase, thereby improving the production efficiency of amino acids of the strain.

## Description

### TECHNICAL FIELD

The present disclosure belongs to the field of biotechnology, and specifically relates to mutants of pyruvate carboxylase gene promoters and applications thereof.

### BACKGROUND

Amino acids, including lysine, threonine, glutamic acid, etc., are basic substances constituting the proteins necessary for animal nutrition, and are widely used in industries such as medicine, health, food, animal fodder, and cosmetics. Amino acids are mainly produced by microbial fermentation. At present, the main production strains include microorganisms such as the genus *Enterobacter* and the genus *Corynebacterium.* Due to the physiological superiority of *Corynebacterium,* it has become the most important production strain in the industry. With the continuous development of the biotechnology, reports on metabolic engineering of *Corynebacterium* to improve its amino acid yield are gradually raising. These engineering modifications include enhancement of the expression of enzymes associated with synthetic pathways of the amino acids, weakening of the expression of enzymes associated with competition pathways, and so forth.

Pyruvate carboxylase (encoded by the *pyc* gene) is thought to be the enzyme that plays a significant role in the anaplerotic pathway of *Corynebacterium,* and this enzyme is capable of catalyzing pyruvic acid and carbon dioxide to produce oxaloacetic acid, thereby entering the TCA cycle. It has been reported in multiple literatures that overexpression of the pyruvate carboxylase can increase the yield of amino acids (such as lysine, glutamic acid, and threonine) of strains ^{[1-2]}. At present, overexpression of the *pyc* gene is mostly achieved by increasing its copy number. However, increasing the copy number may lead to genomic instability of strains, whereas promoters do not have the above deficiency in regulating the gene expression and high-performance promoters can also be used to express different target genes. Therefore, there is a need in the art to develop abundant promoter elements to appropriately enhance the expression of genes such *as pyc,* thereby improving the productivity of the target compound of the strain.

### References:

[1] CN110603321A
[2] CN1275167A

### SUMMARY

In a first aspect, the present disclosure provides a mutant of a pyruvate carboxylase gene promoter, wherein the mutant (i) has one or more mutated nucleotides in a core region corresponding to position 279 to position 317 of a promoter having a nucleotide sequence set forth in SEQ ID NO: 21.

The term "mutant" used herein refers to a polynucleotide or polypeptide that, relative to the "wild-type" or "comparative" polynucleotide or polypeptide, contains alternation(s) (i.e., substitution, insertion and/or deletion of a polynucleotide) at one or more (*e.g.,* several) positions, where the substitution refers to a substitution of a different nucleotide for a nucleotide that occupies one position; the deletion refers to removal of a nucleotide that occupies certain position; and the insertion refers to an addition of a nucleotide after the nucleotide adjacent to and immediately following the occupied position.

Specifically, the mutant of the pyruvate carboxylase gene promoter of *Corynebacterium glutamicum* has mutated nucleotide(s) at 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38 or 39 positions in the nucleotide sequence corresponding to position 279 to position 317 of the nucleotide sequence set forth in SEQ ID NO: 21.

In some embodiments, the mutant comprises a substituted nucleotide. The substitution is a mutation caused by a substitution of one base in the nucleotide with another different base, which is also referred to as a base substitution or a point mutation.

In some embodiments, the mutant (ii) comprises a reverse complementary sequence to the nucleotide sequence set forth in (i).

In some embodiments, the mutant (iii) comprises a reverse complementary sequence to a sequence capable of hybridizing with the nucleotide sequence set forth in (i) or (ii) under high-stringent hybridization conditions or very high-stringent hybridization conditions.

In some embodiments, the mutant (iv) comprises a nucleotide sequence having at least 90% sequence identity to the nucleotide sequence set forth in (i) or (ii). Specifically, the mutant comprises a sequence having at least 80%, 81%, 82%, 83%, 84%, 85%, 86%, 87%, 88%, 89%, 90%, 91%, 92%, 93%, 94%, 95%, 96%, 97%, 98%, or 99% sequence identity to the nucleotide sequence set forth in (i) or (ii).

The nucleotide sequence of the mutant set forth in any one of (i) to (iv) is not CGATGTTTGATTGGGGGAATCGGGGGTTACGATACTAGG at positions corresponding to position 279 to position 317 of the sequence set forth in SEQ ID NO: 21; and preferably, the mutant set forth in any one of (i) to (iv) has an enhanced promoter activity as compared to the pyruvate carboxylase gene promoter having the sequence set forth in SEQ ID NO: 21.

In some embodiments, the mutant of the pyruvate carboxylase gene promoter according to the present disclosure has the following nucleotide sequence corresponding to position 279 to position 317 of the nucleotide sequence set forth in SEQ ID NO: 21: NNNNNNTTGATTNNNNNNNNNNNNNNNTANNATNNNNNN, wherein N is selected from A, T, C, or G.

Preferably, the mutant of the pyruvate carboxylase gene promoter has an enhanced promoter activity of 1 to 17 folds or more as compared to the pyruvate carboxylase gene promoter having the sequence set forth in SEQ ID NO: 21. Exemplarily, the promoter activity after mutation is enhanced by at least 1.8 folds, preferably 3 folds, 5 folds, 8 folds, preferably 10 folds, 11 folds, 12 folds, 13 folds, and more preferably 14 folds, 15 folds, or 16 folds or more.

In some embodiments, the nucleotide sequence of the core region of the promoter of the mutant according to the present disclosure is one of the following sequences:
1) CTAATTTTGATTCGTACTGATTTCTGCTACGATGAGTCA;
2) GGATTGTTGATTTGAGCTTGATGAGCGTACAATCAACTT;
3) TTCTCCTTGATTGCGCCTTAACCGTGGTATGATTCGATA;
4) ATTGATTTGATTGGAACCTTACTGTGCTATGATTTGGTA;
5) TCGAGTTTGATTTCACAACGTGTGTGATAGGATATAATA;
6) TTGCGTTTGATTAAAGTATGCAAGGGCTAGTATGGTGAT;
7) ATCATTTTGATTCCGGCGCACATGTGGTAATATGGTATT;
8) TCGCCATTGATTGCCCGCCATCCATGCTATAATCGGAAG;
9) TTCCGCTTGATTGTGGCCATAGTATGATATTATTAATTA;
10) CGGATCTTGATTTTATGATGGGTATTGTATAATCTTGGT;
11) CGGATATTGATTTGGCCGGTGTTGTGGTAGTATCGTGTT;
12) AGGGGTTTGATTGGCCGCTCGGTGTGTTATCATGGAGAG;
13) GAGTTGTTGATTTCGTTGGTGCACGTATACAATGGTTTT;
14) CTTGGCTTGATTTTTGTTTGAGGGTTGTATAATGTTATT;
15) GACTAGTTGATTTCCGCCCTTGGTTGATATTATGCTTGA;
16) ATCCGCTTGATTTAGGCGTACGTTTAATAGTATATTGAA;
17) CGGGGCTTGATTTCCTTGTCGTGGCGTTATTATAATGGA;
18) ATGGAGTTGATTATACGATACTACAGATACTATACTGGT;
19) CCGTAGTTGATTGACTTGGGCAGTATATAGTATAATGAA; or
20) CGGGCCTTGATTGTAAGATAAGACATTTAGTATAATTAG.

In some embodiments, the mutant of the pyruvate carboxylase gene promoter according to the present disclosure has a nucleotide sequence set forth in any one of SEQ ID NO: 1 to SEQ ID NO: 20.

The mutant of the pyruvate carboxylase gene promoter is a nucleotide molecule having a promoter activity, and has an enhanced activity as compared to the wild-type promoter. The "promoter" refers to a nucleic acid molecule, which is typically located upstream of the coding sequence of a gene of interest, providing a recognition site for the RNA polymerase, and is located upstream of the 5' direction of the start site of mRNA transcription. It is the untranslated nucleic acid sequence to which the RNA polymerase binds to, to initiate transcription of the gene of interest.

The gene encoding the pyruvate carboxylase is the *pyc* gene, which is thought to be the enzyme that plays a significant role in the anaplerotic pathway of *Corynebacterium,* and this enzyme is capable of catalyzing pyruvic acid and carbon dioxide to produce oxaloacetic acid. This is one of the major anaplerotic pathways of the tricarboxylic acid cycle. It has been reported in multiple literatures that overexpression of the pyruvate carboxylase Pyc can increase the yield of amino acids (such as lysine, glutamic acid, and threonine) of strains.

The "promoter core region" refers to a nucleic acid sequence located on a promoter in a prokaryote, which is the core sequence region where the function of the promoter is exerted, mainly including the -35 region, the -10 region, the region between the -35 region and the -10 region, and the transcription starting site, the -35 region is a recognition site of the RNA polymerase, the -10 region is a binding site of the RNA polymerase.

The mutant of the promoter of the present disclosure is an improved promoter of the pyruvate carboxylase gene of *Corynebacterium glutamicum,* which has a higher promoter activity than that of the wild-type promoter, e.g., increasing at least by 1.8 folds, 3 folds, 5 folds, 8 folds, preferably 10 folds, 11 folds, 12 folds, 13 folds, more preferably 14 folds or more, 15 folds or more, 16 folds or more.

The nucleic acid molecule of the promoter of the present disclosure may be isolated or prepared by a standard molecular biology technology. For example, the nucleic acid molecule of the promoter of the present disclosure may be isolated by PCR using suitable primer sequences. Also, the nucleic acid molecule of the promoter of the present disclosure may be prepared by a standard synthetic technique using an automated DNA synthesizer.

The nucleic acid molecules with the promoter activity of the present disclosure may be used for expression of the other gene in *Corynebacterium* or *Enterobacterium,* including, but not limited to, genes associated with synthesis of amino acids, e.g., pyruvate carboxylase Pyc, glutamate dehydrogenase Gdh, aspartate kinase LysC, threonine operon ThrABC, aspartate-semialdehyde dehydrogenase gene Asd, aspartate-ammonia lyase AspB, homoserine dehydrogenase Hom, homoserine O-acetyltransferase MetX, dihydrodipicolinate synthase DapA, dihydrodipicolinate reductase DapB, meso-diaminopimelate dehydrogenase Ddh, glutamate kinase ProB, glutamate-5-semialdehyde dehydrogenase ProA, pyrroline-5-carboxylate dehydrogenase ProC, proline dehydrogenase/pyrroline-5-carboxylate dehydrogenase PutA, 5-aminolevulinic acid synthase HemA, phosphoenolpyruvate carboxylase Ppc, amino acid transport protein, ptsG system-associated protein, pyruvate dehydrogenase AceE, glyceraldehyde-3-phosphate dehydrogenase GapN, or lysine decarboxylase CadA or LdcC, etc.

The strength-based promoter elements of the present disclosure may be used to moderately regulate the expression of the target genes and achieve high-efficient production of the target products.

The term *"Corynebacterium"* used herein refers to microorganisms of the genus *Corynebacterium,* including, but not limited to, *Corynebacterium glutamicum* ATCC 13032, *Corynebacterium glutamicum* ATCC 13869, *Corynebacterium glutamicum* B253, *Corynebacterium glutamicum* ATCC 14067, and derived strains that producing amino acids and prepared from the strains described above.

In a second aspect, the present disclosure provides an expression cassette and a recombinant vector comprising the mutant of the pyruvate carboxylase gene promoter.

The "expression cassette" has the meaning generally understood by a person skilled in the art, *i.e.,* an element containing a promoter and a gene of interest and is capable of expressing the gene of interest.

The term "vector" refers to a DNA construct containing DNA sequences operably ligated to appropriate regulatory sequences, so as to express a gene of interest in a suitable host. The vector used herein is not particularly limited, and may be any vector known in the art as long as it is capable of replicating in a host. That is, the vector includes, but is not limited to, a plasmid and a phage, for example, the pEC-XK99E plasmid used in the specific examples of the present disclosure. Once transformed into a suitable host, the vector may replicate and function independently of the host genome, or integrate into the genome *per se* in some cases.

In a third aspect, the present disclosure provides a recombinant host cell containing the mutant of the pyruvate carboxylase gene promoter, the expression cassette, or the recombinant vector.

The recombinant host cell is specifically achieved by, *e.g.,* transformation. The term "transformation" used herein has the meaning generally understood by a person skilled in the art, *i.e.,* a process of introducing an exogenous DNA into a host. Methods for the transformation include any method for introducing a nucleic acid into a cell. These methods include, but are not limited to, electroporation, calcium phosphate (CaPO₄) precipitation, calcium chloride (CaCl₂) precipitation, microinjection, a polyethylene glycol (PEG) method, a DEAE-dextran method, a cationic liposome method, and a lithium acetate-DMSO method.

The "host cell" used herein has the meaning generally understood by a person skilled in the art, *i.e.,* the cell that can be introduced with the nucleic acid having the promoter activity according to the present disclosure, and is referred to as a recombinant host cell after introduction. In other words, any host cell may be used in the present disclosure as long as it contains the nucleic acid having the promoter activity according to the present disclosure and is operably ligated to a gene to mediate transcription of this gene. The host cell of the present disclosure may be a prokaryotic cell or a eukaryotic cell. In some embodiments, the host cell of the present disclosure may be any types of strain capable of producing the target product, which includes wild-type strains and recombinant strains. Exemplarily, the host cell is derived from microorganisms suitable for fermentation production of target products such as amino acids and organic acids, *e.g., Enterobacterium, Corynebacterium, Brevibacterium, Arthrobacterium,* and *Microbacterium,* etc.

In some preferred embodiments, the host cell is *Enterobacterium* or *Corynebacterium,* more preferably *Corynebacterium glutamicum,* including, but not limited to, *Corynebacterium glutamicum* ATCC 13032, *Corynebacterium glutamicum* ATCC 13869, *Corynebacterium glutamicum* B253, *Corynebacterium glutamicum* ATCC 14067, and L-amino acids-producing derived strains prepared from the strains described above.

In one specific embodiment, the *Corynebacterium glutamicum* is subject to the following modification: a T311I mutation coding sequence is introduced into the coding gene of aspartate kinase in *Corynebacterium glutamicum,* and a gene encoding the pyruvate carboxylase operably ligated to the mutant of the pyruvate carboxylase gene promoter described above is introduced to obtain a lysine-producing strain; or expression cassettes of the glutamate kinase, glutamate-5-semialdehyde dehydrogenase and/or pyrroline-5-carboxylate dehydrogenase, in which a G149K mutation is introduced, operably ligated to the mutant of the pyruvate carboxylase gene promoter according to the present disclosure are integrated into the proline dehydrogenase/pyrroline-5-carboxylate dehydrogenase gene to obtain a proline-producing strain; preferably, the sequence of the core region of the mutant of the pyruvate carboxylase gene promoter is CGGGCCTTGATTGTAAGATAAGACATTTAGTATAATTAG, more preferably, the sequence of the full-length promoter is set forth in SEQ ID NO: 20.

In other embodiments, the host cell may also be other types of amino acids-producing strains. The "amino acids-producing strain" described herein refers to the strain that can produce amino acids when bacteria are cultured in a medium and could accumulate amino acids, or can secrete amino acids into the medium, i.e., extracellular free amino acids are obtainable. For example, the strains may be naturally occurring amino acids-producing strains, or engineered amino acids-producing strains obtained by genetic modifications.

Exemplarily, the host cell is a lysine-producing host cell. In some embodiments, the lysine-producing host cells may further include, but not limited to, one or more genes selected from one or more of the following that are attenuated or reduced in expression:
a. *adhE* gene encoding ethanol dehydrogenase;
b. *ackA* gene encoding acetate kinase;
c. *pta* gene encoding phosphate acetyltransferase;
d. *IdhA* gene encoding lactic dehydrogenase;
e. *focA* gene encoding formate transporter;
f. *pflB* gene encoding pyruvate formate lyase;
g. *poxB* gene encoding pyruvate oxidase;
h. *thrA* gene encoding aspartate kinase I/homoserine dehydrogenase I bifunctional enzyme;
i. *thrB* gene encoding homoserine kinase;
j. *IdcC* gene encoding lysine decarboxylase; and
h. *cadA* gene encoding lysine decarboxylase.

In some embodiments, the lysine-producing host cells may further include, but not limited to, one or more genes selected from one or more of the following that are enhanced or overexpressed:
a. *dapA* gene encoding dihydrodipyridine synthase that relieves feedback inhibition of lysine;
b. *dapB* gene encoding dihydrodipicolinate reductase;
c. *ddh* gene encoding diaminopimelate dehydrogenase;
d. *dapD* encoding tetrahydrodipicolinate succinylase and *dapE* encoding succinyldiaminopimelate deacylase;
e. *asd gene* encoding aspartate-semialdehyde dehydrogenase;
f. *ppc* gene encoding phosphoenolpyruvate carboxylase;
g. *pntAB* gene encoding niacinamide adenine dinucleotide transhydrogenase; and
i. *lysE* gene encoding transport protein of lysine.

Exemplarily, the host cells are threonine-producing host cells. In some embodiments, the threonine-producing host cells may be strains expressing the aspartate kinase LysC that relieves the feedback inhibition based on the *Corynebacterium glutamicum* ATCC 13032. In other embodiments, the threonine-producing host cells may also be other types of strains having the capability of producing threonine.

In some embodiments, one or more genes selected from the group consisting of the following genes in the threonine-producing host cells are enhanced or overexpressed:
a. *thrABC* gene encoding threonine operon;
b. *hom* gene encoding homoserine dehydrogenase that relieves the feedback inhibition;
c. *gap* gene encoding glyceraldehyde-3-phosphate dehydrogenase;
d. *pyc* gene encoding pyruvate carboxylase;
e. *mqo* gene encoding malate : quinone oxidoreductase;
f. *tkt* gene encoding transketolase;
g. *gnd* gene encoding 6-phosphogluconate dehydrogenase;
h. *thrE* gene encoding threonine exporter; and
i. *eno* gene encoding enolase.

Exemplarily, the host cells are isoleucine-producing host cells. In some embodiments, the isoleucine-producing host cells are strains that produce L-isoleucine by substituting alanine for the amino acid at position 323 of the *ilvA* gene in L-threonine dehydratase. In other embodiments, the isoleucine-producing host cells may also be other types of strains having the isoleucine-producing capability.

Exemplarily, the host cells are O-acetylhomoserine-producing host cells. In some embodiments, the O-acetylhomoserine-producing host cells are strains that produce O-acetylhomoserine by inactivating O-acetylhomoserine(thiol)-lyase. In other embodiments, the O-acetylhomoserine-producing host cells may also be other types of strains having the O-acetyl homo serine-producing capability.

Exemplarily, the host cells are methionine-producing host cells. In some embodiments, the methionine-producing host cells are strains that produce methionine by inactivating the transcriptional regulators of methionine and cysteine. In other embodiments, the methionine-producing host cells may also be other types of strains having the methionine-producing capability.

In the present disclosure, the host cells may be cultured by conventional methods in the art, including, but not limited to, well-plate culture, shaking culture, batch culture, continuous culture, fed-batch culture, etc. Also, various culture conditions such as the temperature, time, and pH value of the medium may be properly adjusted according to actual situations.

In a fourth aspect, the present disclosure provides use of the mutant of the pyruvate carboxylase gene promoter according to the first aspect, the expression cassette or expression vector according to the second aspect, or the recombinant host cell according to the third aspect in at least one of the following (a) to (c):
(a) enhancing the transcriptional level of a gene, or preparing a reagent or kit for enhancing the transcriptional level of a gene;
(b) preparing a protein, or preparing a reagent or kit for use in the preparation of a protein; and
(c) producing a target product, or preparing a reagent or a kit for use in the production of a target product.

In some embodiments, the protein is a target product synthesis-associated protein, a membrane transport-associated protein, or a gene expression regulatory protein.

In some embodiments, the target product may be selected from at least one amino acids and derivatives thereof, or may be selected from other kinds of compounds that may be biosynthetically available in the art.

Exemplarily, the amino acids and derivatives thereof include, but are not limited to, one or a combination of two or more s of the following: proline, hydroxyproline, lysine, glutamic acid, arginine, ornithine, glutamine, threonine, glycine, alanine, valine, leucine, isoleucine, serine, cysteine, methionine, aspartic acid, asparagine, histidine, phenylalanine, tyrosine, tryptophan, 5-aminolevulinic acid, 5-aminovaleric acid, or derivatives of any one of the amino acids described above.

In a fifth aspect, the present disclosure provides a method for enhancing expression of a gene of interest, the method comprising operably ligating the mutant of the pyruvate carboxylase gene promoter to the gene of interest, preferably enhancing expression of a gene associated with synthesis of a target product using oxaloacetic acid as a precursor.

The term "operably ligated" used herein means that the mutant of the pyruvate carboxylase gene promoter of the present disclosure is functionally ligated to the coding gene to initiate and mediate transcription of the gene, indicating that the mutant of the pyruvate carboxylase gene promoter of the present disclosure is operably ligated to the coding gene to control the transcriptional activity of the operon gene. The methods of the operable ligating may be any method described in the art. In case of using the mutant of the pyruvate carboxylase gene promoter according to the present disclosure, the method of enhancing expression of a target gene according to the present disclosure includes the methods commonly adopted by a person skilled in the art.

Exemplarily, the coding gene includes, but is not limited to, a coding gene of a target product synthesis-associated protein, a coding gene of a membrane transport-associated protein, or a coding gene of a gene expression regulatory protein.

In some embodiments, the mutant of the pyruvate carboxylase gene promoter is used for expression of the gene associated with synthesis of amino acids. Exemplarily, the mutant of the pyruvate carboxylase gene promoter may be used for expression of the other genes in *Corynebacterium* or *Enterobacterium,* including, but not limited to, genes associated with synthesis of amino acids, e.g., glutamate dehydrogenase Gdh, pyruvate carboxylase gene Pyc, aspartate kinase LysC, threonine operon ThrABC, aspartate-semialdehyde dehydrogenase Asd, aspartate-ammonia lyase AspB, homoserine dehydrogenase Hom, homoserine O-acetyltransferase MetX, dihydrodipicolinate synthase DapA, dihydrodipicolinate reductase DapB, meso-diaminopimelate dehydrogenase Ddh, glutamate kinase ProB, glutamate-5-semialdehyde dehydrogenase ProA, pyrroline-5-carboxylate dehydrogenase ProC, proline dehydrogenase/pyrroline-5-carboxylate dehydrogenase PutA, glutamyl-t-RNAreductase HemA, phosphoenolpyruvate carboxylase Ppc, amino acid transport protein, ptsG system-associated protein, pyruvate dehydrogenase AceE, glyceraldehyde-3-phosphate dehydrogenase GapN, or lysine decarboxylase CadA or LdcC, etc.

In other embodiments, the mutant of the pyruvate carboxylase gene promoter is used for expression of the gene associated with synthesis of organic acids, for example, proteins associated with synthesis of citric acid, or genes for encoding proteins associated with synthesis of succinic acid.

In a sixth aspect, the present disclosure provides a method for preparing a protein, wherein the method comprises a step of expressing the protein using the expression cassette or expression vector according to the second aspect, or the recombinant host cell according to the third aspect; optionally, the protein is a target product synthesis-associated protein, a membrane transport-associated protein, or a gene expression regulatory protein; and
optionally, the method further comprises a step of isolating or purifying the protein.

In a seventh aspect, the present disclosure provides a method for producing a target product, wherein the method comprises culturing a host cell containing the mutant of the pyruvate carboxylase gene promoter according to the first aspect that is operably ligated to a gene associated with synthesis of the target product, and collecting the resulting target product.

Furthermore, the present disclosure provides a method for producing a target product with oxaloacetic acid as a precursor, the method comprising culturing a host cell containing the mutant of the pyruvate carboxylase gene promoter that is operably ligated to a gene associated with synthesis of the target product with the oxaloacetic acid as the precursor, and collecting the resulting target product. Wherein, the target product with the oxaloacetic acid as the precursor includes, but is not limited to, amino acids and derivatives thereof. The amino acids include amino acids of the aspartic acid family (lysine, threonine, isoleucine, and methionine), amino acids of the glutamic acid family (glutamic acid, proline, hydroxyproline, arginine, and glutamine), and 5-aminolevulinic acid, etc. The derivatives include, but are not limited to, pentanediamine, glutaric acid, etc. With the methods disclosed herein, the yield of the target products with oxaloacetic acid as a precursor can be increased in strains. More specifically, the gene associated with synthesis of the target product with the oxaloacetic acid as the precursor is selected from the group consisting of: the *pyc* gene, the *gdh* gene, the *lysC* gene, the *thrABC* gene, the *asd* gene, the *aspB* gene, the *hom* gene, the *metX* gene, the *dapA* gene, the *dapB* gene, the *ddh* gene, the *proB* gene, the *proA* gene, the *proC* gene, the *putA* gene, the *hemA* gene, the *ppc* gene, the *lysE* gene, the *ptsG* gene, the *ptsI* gene, the *aceE* gene, the *gapN* gene, the *cadA* gene, the *IdcC* gene, etc.

The method for producing the target product with oxaloacetic acid as a precursor according to the present disclosure is the method commonly used by a person skilled in the art on the basis of using the mutant of the pyruvate carboxylase gene promoter, and meanwhile the method comprises a step of recovering a target product from a cell or a culture solution. The method for recovering the target product from the cell or medium is well-known in the art, and includes, but is not limited to, filtration, anion exchange chromatography, crystallization, and HPLC.

The present disclosure achieves the following advantageous effects: the nucleic acid molecule with an enhanced promoter activity provided herein exhibits a higher promoter activity than that of a wild type, and is useful for expression of the target gene, in particular the expression of the gene associated with production of amino acids. For example, when operably ligated to the target product synthesis-associated gene such as the *pyc* gene can enhance the expression intensity of target product synthesis-associated proteins such as the pyruvate carboxylase, thereby improving the production efficiency of amino acids of the recombinant strains and derivatives thereof, etc., so it has a relatively high application value.

### BRIEF DESCRIPTION OF THE DRAWINGS

FIG. 1: Plasmid vector map of pEC-XK99E-P*pyc*-*rfp* plasmid.
FIG. 2: Fluorescent screening plate of *pyc* promoter mutants.

### DETAILED DESCRIPTION

### Definitions of Terms

When used in combination with the term "including" in the claims and/or specification, the word "a" or "an" may refer to "one", or refer to "one or more", "at least one", and "one or more than one".

As used in the claims and specification, the term "including", "having", "comprising", or "containing" is intended to be inclusive or open-ended, and does not exclude additional or unrecited elements or methods and steps.

Throughout the application document, the term "about" means that a value includes the error or standard deviation caused by the device or method used to measure the value.

It is applicable to the content disclosed herein that the term "or" is defined only as alternatives and "and/or", but the term "or" used herein refers to "and/or" unless otherwise expressly stated to be only alternatives or mutual exclusion between alternatives.

The selected/optional/preferred "numerical range", when used in the claims or the specification, includes not only the numerical endpoints at both ends of the range, but also all natural numbers covered between the above numerical endpoints with respect to these numerical endpoints.

As used herein, the term "nucleic acid molecule" or "polynucleotide" refers to a polymer composed of nucleotides. A nucleic acid molecule or polynucleotide may be in the form of an individual fragment, or may be a constituent part of a larger nucleotide sequence structure, which is derived from the nucleotide sequence that has been isolated at least once in number or concentration, and could be recognized, operated, and sequence recovered as well as nucleotide sequence recovered by a standard molecular biological method (*e.g.,* using a cloning vector). When a nucleotide sequence is represented by a DNA sequence (*i.e.,* A, T, G, C), it also includes an RNA sequence (*i.e.,* A, U, G, C), where "U" substitutes for "T". In other words, "polynucleotide" refers to a nucleotide polymer knocked out from an additional nucleotide (an individual fragment or an entire fragment), or may be a constituent part or component of a larger nucleotide structure, such as an expression vector or a polycistronic sequence. Polynucleotides include DNA, RNA, and cDNA sequences.

As used herein, the terms "target gene" and "gene of interest" can be used interchangeably.

As used herein, the term "wild-type" refers to an object that can be found in nature. For example, a polypeptide or a polynucleotide sequence present in an organism that can be isolated from one source in nature and has not been intentionally modified by human in the laboratory is naturally occurring. As used herein, "naturally occurring" and "wild-type" are synonymous.

As used herein, the terms "sequence identity" and "percent identity" refer to the percentage of nucleotides or amino acids that are the same (*i.e.,* identical) between two or more polynucleotides or polypeptides. The sequence identity between two or more polynucleotides or polypeptides may be determined by aligning the nucleotide sequences of polynucleotides or the amino acid sequences of polypeptides and scoring the number of positions at which nucleotide or amino acid residues are identical in the aligned polynucleotides or polypeptides, and comparing the number of these positions with the number of positions at which nucleotide or amino acid residues are different in the aligned polynucleotides or polypeptides. Polynucleotides may differ at one position by, *e.g.,* containing a different nucleotide (*i.e.,* substitution or mutation) or deleting a nucleotide (*i.e.,* insertion or deletion of a nucleotide in one or two polynucleotides). Polypeptides may differ at one position by, *e.g.,* containing a different amino acid (i.e., substitution or mutation) or deleting an amino acid (*i.e.,* insertion or deletion of an amino acid in one or two polypeptides). The sequence identity may be calculated by dividing the number of positions at which nucleotide or amino acid residues are identical by the total number of nucleotide or amino acid residues in the polynucleotides or polypeptides. For example, the percent identity may be calculated by dividing the number of positions at which nucleotide or amino acid residues are identical by the total number of nucleotide or amino acid residues in the polynucleotides or polypeptides, and multiplying the result by 100.

In some embodiments, two or more sequences or subsequences, when compared and aligned at maximum correspondence by the sequence alignment algorithm or by the visual inspection measurement, have at least 80%, 81%, 82%, 83%, 84%, 85%, 86%, 87%, 88%, 89%, 90%, 91%, 92%, 93%, 94%, 95%, 96%, 97%, 98%, or 99% "sequence identity" or "percent identity" of nucleotides. In some embodiments, the sequence is substantially identical over the full length of either or both of the compared biopolymers (*e.g.,* polynucleotides).

As used herein, the term "complementary" refers to hybridization or base pairing between nucleotides or nucleotides, *e.g.,* between two chains of a double-stranded DNA molecule or between an oligonucleotide primer and a primer binding site on a single-stranded nucleotide to be sequenced or amplified.

As used herein, the term "high-stringent conditions" means that following the standard DNA blotting procedures, a probe of at least 100 nucleotides in length pre-hybridizes or hybridizes for 12 to 24 hours at 42°C in 5X SSPE (saline sodium phosphate EDTA), 0.3% SDS, 200 µg/ml of cleavaged and denatured salmon sperm DNA, and 50% formamide. Finally, the vector material is washed three times at 65°C with 2X SSC and 0.2% SDS, each for 15 min.

As used herein, the term "very high-stringent conditions" means that following the standard DNA blotting procedures, a probe of at least 100 nucleotides in length pre-hybridizes or hybridizes for 12 to 24 hours at 42°C in 5X SSPE (saline sodium phosphate EDTA), 0.3% SDS, 200 µg/ml of cleavaged and denatured salmon sperm DNA, and 50% formamide. Finally, the vector material is washed three times at 70°C with 2X SSC and 0.2% SDS, each for 15 min.

Unless otherwise defined, all technical and scientific terms used herein have the same meanings as typically understood by one of ordinary skill in the art to which the present disclosure belongs. Although any methods and materials similar or equivalent to those described herein may be used to implement or test the present disclosure, the methods and materials described herein are preferred.

The present disclosure will be further illustrated with reference to specific examples. It should be appreciated that these examples are merely intended to illustrate the present disclosure and are not intended to limit the scope of the present disclosure. The experimental techniques and experimental methods used in the examples, unless otherwise specified, are all conventional techniques and methods, for example, experimental methods for which no specific conditions are indicated in the following examples are generally performed according to conventional conditions such as those described by Sambrook et al., Molecular Cloning: A Laboratory Manual (New York: Cold Spring Harbor Laboratory Press, 1989), or those recommended by manufacturers. The materials, reagents, etc. used in the examples, unless otherwise specified, are all commercially available.

### Example 1. Construction of plasmid characterizing the strength of pyc gene promoter of Corynebacterium glutamicum

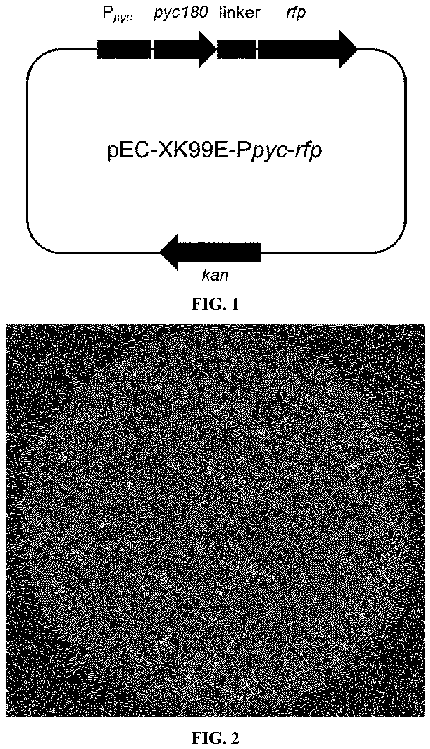

In order to characterize the strength of the *pyc* gene promoter of *Corynebacterium glutamicum,* the present disclosure firstly constructed a characterizing vector, and based on the skeleton of the pEC-XK99E plasmid, 60 amino acids at the N-terminus of the *pyc* gene, a C-peptide, and a red fluorescence protein gene were expressed by the *pyc* gene promoter. Based on the disclosed genome sequence and *pyc* gene annotation information of *Corynebacterium glutamicum* ATCC13032, pyc-FIR primers were designed, and a 180-bp DNA fragment of the *pyc* gene promoter and the N-terminus was obtained by PCR amplification using the ATCC 13032 genome as a template. The pEC-XK99E-*rjp*^{[3]} plasmid reported in the literature was used as a template and pEC-F/R as primers to amplify the DNA fragment of the skeleton of the pEC-XK99E plasmid, the C-peptide, and the red fluorescence protein gene. The above two fragments were ligated via Vazyme's One Step Cloning Kit to obtain a pEC-XK99E-P*pyc*-*rfp* characterizing vector. The plasmid vector map was as shown in FIG. 1. The sequences of the primers as used above were listed in Table 1.

**Table 1**

| Primer | Nucleotide Sequence | SEQ ID NO. |
|---|---|---|
| *pyc-*F | CCTGATGCGGTATTTTCTCCGAAAACCCAGGATTGCTTT GTG | SEQ ID NO: 22 |
| *pyc-*R | GCGGACAGCTTCAGAAGCAAAAG | SEQ ID NO: 23 |
| pEC-F | TTGCTTCTGAAGCTGTCCGCGGCGGTGGCTCTGGAGGT GGTGGGTCCGGCGGTGGCTCTGCTTCCTCCGAAGACGT TATCAAAG | SEQ ID NO: 24 |
| pEC-R | GGAGAAAATACCGCATCAGGC | SEQ ID NO: 25 |

### Example 2. Screening and strength characterization of pyc gene promoter mutants of Corynebacterium glutamicum

### (1) Construction of library of pyc gene promoter mutants of Corynebacterium glutamicum

The present disclosure performed mutations on the core region of the *pyc* gene promoter of *Corynebacterium glutamicum:*
"CGATGTTTGATTGGGGGAATCGGGGGTTACGATACTAGG", where the underlined parts were the main sequences of the -35 region and the -10 region of the promoter. The present disclosure performed mutations on the corresponding positions of the above core region, obtaining "NNNNNNTTGATTNNNNNNNNNNNNNNNTANNATNNNNNN". The two fragments of the plasmid were amplified using *pyc*-M1/M2 and *pyc*-M3/M4 primers respectively, and ligated via Vazyme's One Step Cloning Kit. All of the resulting cloned bacteria were collected and plasmids were extracted to obtain a library of the *pyc* gene promoter mutants. The above library and the wild-type control pEC-XK99E-P*pyc*-*rfp* obtained in Example 1 were transformed into the *Corynebacterium glutamicum* ATCC13032 respectively, and coated on a TSB plate. The plate, on which hundreds of clones were grown, was fluorescence photographed with a fluorescence imaging system. Mutants with improved expression intensity were preliminarily screened according to the fluorescence brightness of the clones. The ingredients (g/L) of the medium of the TSB plate were as follows: glucose, 5 g/L; yeast powder, 5 g/L; soy peptone, 9 g/L; urea, 3 g/L; succinic acid, 0.5 g/L; K₂HPO₄·3H₂O, 1 g/L; MgSO₄·7H₂O, 0.1 g/L; biotin, 0.01 mg/L; vitamin B1, 0.1 mg/L; MOPS, 20 g/L; and agar powder, 15 g/L. The present disclosure performed initial screening on more than 10,000 clones, and as shown in FIG. 2, about 30 mutants with enhanced fluorescence intensity were obtained. The sequences of the primers as used above were listed in Table 2.

**Table 2**

| Primer | Nucleotide Sequence | SEQ ID NO. |
|---|---|---|
| *pyc*-M1 | CCCGAAAACATTGAGAGGAAAACAAAAACNNNNNNTTG ATTNNNNNNNNNNNNNNNTANNATNNNNNNACGCAGTG ACTGCTATCACCC | SEQ ID NO: 26 |
| *pyc-*M2 | AACCTTCCATACGAACTTTGAAACG | SEQ ID NO: 27 |
| *pyc-*M3 | CAAAGTTCGTATGGAAGGTTCCG | SEQ ID NO: 28 |
| *pyc-*M4 | TTCCTCTCAATGTTTTCGGGC | SEQ ID NO: 29 |

### (2) Screening of library of pyc gene promoter mutants of Corynebacterium glutamicum

All mutants with the enhanced fluorescence intensity observed in the plate as described above were cultured in a 96-well plate to characterize the strength of the promoters. The ingredients (g/L) of the TSB liquid medium were as follows: glucose, 5 g/L; yeast powder, 5 g/L; soy peptone, 9 g/L; urea, 3 g/L; succinic acid, 0.5 g/L; K₂HPO₄·3H₂O, 1 g/L;MgSO₄·7H₂O, 0.1 g/L; biotin, 0.01 mg/L; vitamin B1, 0.1 mg/L; and MOPS, 20 g/L. The strains resulting from the plate were inoculated with toothpicks into a 96-well plate containing 200 µl of TSB liquid medium in each well. Three samples were set for each strain. The rotating speed of the plate shaker was 800 rpm. After culturing at 30°C for 24 h, the fluorescence intensities of the strains were detected, and the strains with improved fluorescence intensity as compared to the wild-type control were sequenced. The results were listed in Table 3. Some of the promoter mutants have the same sequence. Finally, the present disclosure successfully obtained 20 different promoter mutants (the nucleotide sequences of the corresponding mutated promoters were numbered as SEQ ID NOS: 1 to 20) with improved expression intensity as compared to the wild-type promoter, and the range of the increased folds was from 1.8 folds to 16.1folds, which could provide abundant elements for modifying the expression of genes such as *pyc.*

**Table 3**

| Promoter No. | Fluorescenc e Intensity (RFU/OD₆₀₀ ) | Folds Increased Than Wild-Type | Core Region Sequence of Promoter | SEQ ID NO. of Complete Sequence of Promoter |
|---|---|---|---|---|
| WT | 208±3 | | CGATGTTTGATTGGGGGAATCGGGGGTTACGATACTA GG | SEQ ID NO: 21 |
| P*_{pyc}*-1 | 578±13 | 1.8 | CTAATTTTGATTCGTACTGATTTCTGCTACGATGAGTC A | SEQ ID NO: 1 |
| P*_{pyc}*-2 | 822±5 | 3.0 | GGATTGTTGATTTGAGCTTGATGAGCGTACAATCAAC TT | SEQ ID NO: 2 |
| *P_{pyc}*-3 | 833±7 | 3.0 | TTCTCCTTGATTGCGCCTTAACCGTGGTATGATTCGA TA | SEQ ID NO: 3 |
| *P_{pyc}*-4 | 1185±34 | 4.7 | ATTGATTTGATTGGAACCTTACTGTGCTATGATTTGG TA | SEQ ID NO: 4 |
| P*_{pyc}*-5 | 1203±27 | 4.8 | TCGAGTTTGATTTCACAACGTGTGTGATAGGATATAA TA | SEQ ID NO: 5 |
| P*_{pyc}*-6 | 1208±28 | 4.8 | TTGCGTTTGATTAAAGTATGCAAGGGCTAGTATGGTG AT | SEQ ID NO: 6 |
| P*_{pyc}*-7 | 1495±46 | 6.2 | ATCATTTTGATTCCGGCGCACATGTGGTAATATGGTAT T | SEQ ID NO: 7 |
| P*_{pyc}*-8 | 1498±23 | 6.2 | TCGCCATTGATTGCCCGCCATCCATGCTATAATCGGA AG | SEQ ID NO: 8 |
| P*_{pyc}*-9 | 1544±25 | 6.4 | TTCCGCTTGATTGTGGCCATAGTATGATATTATTAATT A | SEQ ID NO: 9 |
| P*_{pyc}*-10 | 1776±127 | 7.5 | CGGATCTTGATTTTATGATGGGTATTGTATAATCTTGG T | SEQ ID NO: 10 |
| P*_{pyc}*-11 | 1819±14 | 7.7 | CGGATATTGATTTGGCCGGTGTTGTGGTAGTATCGTG TT | SEQ ID NO: 11 |
| P*_{pyc}*-12 | 1834±67 | 7.8 | AGGGGTTTGATTGGCCGCTCGGTGTGTTATCATGGA GAG | SEQ ID NO: 12 |
| P*_{pyc}*-13 | 2066±15 | 8.9 | GAGTTGTTGATTTCGTTGGTGCACGTATACAATGGTT TT | SEQ ID NO: 13 |
| P*_{pyc}*-14 | 2248±142 | 9.8 | CTTGGCTTGATTTTTGTTTGAGGGTTGTATAATGTTAT T | SEQ ID NO: 14 |
| P*_{pyc}*-15 | 2305±87 | 10.1 | GACTAGTTGATTTCCGCCCTTGGTTGATATTATGCTTG A | SEQ ID NO: 15 |
| P*_{pyc}*-16 | 2725±160 | 12.1 | ATCCGCTTGATTTAGGCGTACGTTTAATAGTATATTGA A | SEQ ID NO: 16 |
| P*_{pyc}*-17 | 2987±103 | 13.4 | CGGGGCTTGATTTCCTTGTCGTGGCGTTATTATAATG GA | SEQ ID NO: 17 |
| P*_{pyc}*-18 | 3093±159 | 13.9 | ATGGAGTTGATTATACGATACTACAGATACTATACTG GT | SEQ ID NO: 18 |
| P*_{pyc}*-19 | 3382±211 | 15.3 | CCGTAGTTGATTGACTTGGGCAGTATATAGTATAATG AA | SEQ ID NO: 19 |
| P*_{pyc}*-20 | 3560±202 | 16.1 | CGGGCCTTGATTGTAAGATAAGACATTTAGTATAATT AG | SEQ ID NO: 20 |

### Example 3. Application of pyc gene promoter mutants of Corynebacterium glutamicum of production of target products

### (1) Construction of recombinant vector of pyc gene promoter mutant of Corynebacterium glutamicum

Based on the reported genome sequence of *Corynebacterium glutamicum* ATCC13032, the upstream and downstream homologous arms of the P*_{pyc}*-1, P*_{pyc}*-9, P*_{pyc}*-16, and P*_{pyc}*-20 promoter mutants were subjected to PCR amplification using the ATCC13032 genome as a template and using *pyc*-UF/ *pyc*-UR1 and *pyc-DF1*/ *pyc-DR, pyc*-UF/ *pyc-UR9* and *pyc-DF9*/ *pyc-DR, pyc-*UF/ pyc-UR16 and *pyc-DF161 pyc-DR,* and *pyc*-UF/ *pyc-UR20* and *pyc-DF20*/ *pyc-DR* as primers, respectively; and at the same time, the pK18mobsacB skeleton was amplified using pK18-1/2 as primers. The above PCR fragments were recovered and then ligated via Vazyme's One Step Cloning Kit, and recombinant vectors pK18- *P_{pyc}*-1, pK18- P*_{pyc}*-9, pK18- P*_{pyc}*-16, and pK18- P*_{pyc}*-20 with mutated promoters were obtained, respectively. The sequences of the primers as used above were listed in Table 4.

**Table 4**

| Primer | Nucleotide Sequence | SEQ ID NO. |
|---|---|---|
| *pyc*-UF | CAGGAAACAGCTATGACATGGTATCGCCATGTATCACGCACTC | SEQ ID NO: 30 |
| *pyc*-UR1 | TCAGTACGAATCAAAATTAGGTTTTTGTTTTCCTCTCAATGTTTTC | SEQ ID NO: 31 |
| *pyc*-UR9 | ATGGCCACAATCAAGCGGAAGTTTTTGTTTTCCTCTCAATGTTTTC | SEQ ID NO: 32 |
| *pyc*-UR16 | TACGCCTAAATCAAGCGGATGTTTTTGTTTTCCTCTCAATGTTTTC | SEQ ID NO: 33 |
| *pyc*-UR20 | TATCTTACAATCAAGGCCCGGTTTTTGTTTTCCTCTCAATGTTTTC | SEQ ID NO: 34 |
| *pyc*-DF1 | CTAATTTTGATTCGTACTGATTTCTGCTACGATGAGTCAACGCAGTGA CTGCTATCACCC | SEQ ID NO: 35 |
| *pyc*-DF9 | TTCCGCTTGATTGTGGCCATAGTATGATATTATTAATTAACGCAGTGAC TGCTATCACCC | SEQ ID NO: 36 |
| *pyc*-DF16 | ATCCGCTTGATTTAGGCGTACGTTTAATAGTATATTGAAACGCAGTGA | SEQ ID NO: 37 |
| | CTGCTATCACCC | |
| *pyc*-DF20 | CGGGCCTTGATTGTAAGATAAGACATTTAGTATAATTAGACGCAGTGA CTGCTATCACCC | SEQ ID NO: 38 |
| *pyc*-DR | TGTAAAACGACGGCCAGTGCCTAATTTGCGAAGCTCATCAGGTG | SEQ ID NO: 39 |
| pK18-1 | GCACTGGCCGTCGTTTTAC | SEQ ID NO: 40 |
| pK18-2 | CATGTCATAGCTGTTTCCTGTGTG | SEQ ID NO: 41 |

### (2) Construction of pyc gene promoter mutants of lysine-producing strain of Corynebacterium glutamicum

The recombinant vectors pK18- P*_{pyc}*-1, pK18- P*_{pyc}*-9, pK18- P*_{pyc}*-16, and pK18- P*_{pyc}*-20 as constructed above were transformed into the lysine-producing strain SCgL30 (in which Thr at position 311 of the aspartate kinase of *Corynebacterium glutamicum* ATCC13032 was mutated as Ile ^{[4]}) of *Corynebacterium glutamicum,* respectively. The strain was coated on LBHIS solid media containing 5 g/L of glucose and 25 µg/mL of kanamycin and cultured at 30°C to obtain the first recombinant transformants. The correct first recombinant transformants were inoculated into LB media containing 5 g/L of glucose respectively, and cultured overnight. Thereafter, 1% of the culture was transferred into an LB medium containing 100 g/L of sucrose and cultured overnight at 30°C, and then coated respectively on LB solid media containing 100 g/L of sucrose for screening. Sequencing was performed for confirmation to obtain strains SCgL33, SCgL34, SCgL35, and SCgL36 with the mutated *pyc* promoters, respectively.

### (3) Evaluation of lysine productivity of pyc gene promoter mutants in lysine-producing strain of Corynebacterium glutamicum

To test the effect of applying the mutation of the *pyc* promoter in *Corynebacterium glutamicum* on production of lysine by strains, fermentation tests were performed in SCgL30, SCgL33, SCgL34, SCgL35, and SCgL36, respectively. The ingredients of the fermentation medium were as follows: glucose, 80 g/L; yeast powder, 1 g/L; soy peptone, 1 g/L; NaCl, 1 g/L; ammonium sulfate, 1 g/L; urea, 8 g/L; K₂HPO₄·3H₂O, 1 g/L; MgSO₄·7H₂O, 0.45 g/L; FeSO4·7H₂O, 0.05 g/L; biotin, 0.4 mg/L; vitamin B1, 0.1 mg/L; MOPS, 40 g/L; and initial pH7.2. The strains were firstly inoculated into TSB liquid media and cultured for 8 h. The cultures were inoculated as seeds into a 24-well plate containing 800 µl of fermentation media in each well with the initial OD₆₀₀ controlled at about 0.1, and cultured at 30°C for 20 h. The rotating speed of the plate shaker was 800 rpm. Three samples were set for each strain. After the fermentation, the lysine yields were measured. The results were listed in Table 5. The lysine yields of the strains after mutation of the *pyc* promoter were all significantly increased, and the increase was more significant as the promoter strength was enhanced.

**Table 5**

| Strain | Lysine Yield (g/L) |
|---|---|
| SCgL30 | 1.67±0.03 |
| SCgL33 | 1.77±0.04 |
| SCgL34 | 2.07±0.06 |
| SCgL35 | 2.37±0.15 |
| SCgL36 | 3.27±0.15 |

### Example 4. Application of pyc gene promoter mutants of Corynebacterium glutamicum for proline production

### (1) Application of P_{pyc}-20 in construction of proline-producing strains

In the present disclosure, the G149K mutation was introduced in the *Corynebacterium glutamicum* ATCC13032 strain, and the codon was mutated from GGT to AAG, thereby obtaining a SLCgP1 strain. The SLCgP1 strain was further applied in the present disclosure to integrate the expression cassette, which was over-expressed using the P*_{pyc}*-20 promoter, of glutamate kinase *proB*^{G149K} which relieves the feedback inhibition, glutamate-5-semialdehyde dehydrogenase *proA* along with pyrroline-5-carboxylate dehydrogenase*proC* into the *putA* gene, to obtain a proline-producing strain designated as a SLCgP2 strain.

The SLCgP2 strain was specifically constructed as follows: 1) Firstly, constructing the P*_{pyc}*-20 promoter on the pEC-XK99E plasmid to express the expression cassette of *proB*^{G149K}, *proA,* along with *proC.* The P*_{pyc}*-20 promoter fragment was amplified using the genome of the SCgL36 strain as a template and pyc-a/b as primers; the fragments of *proB*^{G149K}, *proA,* and *proC* were amplified using the genome of the SLCgP1 strain as a template and *proB-*1/2*, proA-*1/2*,* and *proC-1*/*2* as primers, respectively; and at the same time, the pEC-XK99E skeleton was amplified using pEC-1/2 as primers. The above 5 fragments were ligated via Vazyme's One Step Cloning Kit to obtain a pEC- *proB*^{G149K}*proAproC* plasmid. 2) Constructing a recombinant vector comprising the above expression cassette inserted into the *putA* gene on the chromosome. The upstream and downstream homologous arms inserted into the *putA* gene were amplified using the genome of the SCgL36 strain as a template and *putA-*1/2 and *putA-*3/4 as primers, respectively; the expression cassette fragment was amplified using the pEC*-proB*^{G149K}*proAproC* plasmid as a template and *ABC*-F/R as primers; and at the same time, the pK18mobsacB skeleton was amplified using pK18-1/2 as primers. The above PCR fragments were ligated via Vazyme's One Step Cloning Kit to obtain a pK18-*proB*^{G149K}*proAproC* recombinant vector. 3) The pK18-*proB*^{G149K}*proAproC* recombinant vector was transformed into the SLCgP1 strain. The strain was coated on an LBHIS solid medium containing 5 g/L of glucose and 25 µg/mL of kanamycin and cultured at 30°C to obtain the first recombinant transformants. The correct first recombinant transformants were inoculated into LB media containing 5 g/L of glucose respectively, and cultured overnight. Thereafter, 1% of the culture was transferred into an LB medium containing 100 g/L of sucrose and cultured overnight at 30°C, and then coated respectively on an LB solid medium containing 100 g/L of sucrose for screening. Correct mutants were confirmed by PCR with universal and specific primers and sequencing to obtain SLCgP2 strains, respectively. The sequences of the primers as used above were listed in Table 6.

**Table 6**

| Primer | Nucleotide Sequence | SEQ ID NO. |
|---|---|---|
| pEC-1 | GGAGAAAATACCGCATCAGGC | SEQ ID NO: 42 |
| pEC-2 | CTGTTTTGGCGGATGAGAGAAG | SEQ ID NO: 43 |
| *pyc*-a | CCTGATGCGGTATTTTCTCCGAAAACCCAGGATTGCTTT GTG | SEQ ID NO: 44 |
| *pyc-*b | TTGGAGATGCGCTCACGCATTAGAGTAATTATTCCTTTC AACAAGAG | SEQ ID NO: 45 |
| *proB-*1 | ATGCGTGAGCGCATCTCCAAC | SEQ ID NO: 46 |
| *proB-*2 | TTACGCGCGGCTGGCGTAGTTG | SEQ ID NO: 47 |
| *proA*-1 | ACTACGCCAGCCGCGCGTAAGCCTTTTATGGTGTGATCC GAC | SEQ ID NO: 48 |
| *proA-*2 | TTAAGGCCTAATTTGTCCTGTGCC | SEQ ID NO: 49 |
| *proC-*1 | CAGGACAAATTAGGCCTTAATTTGTCGTTTTGGGCCCCC | SEQ ID NO: 50 |
| *proC-*2 | TCTCTCATCCGCCAAAACAGCTAGCGCTTTCCGAGTTCT TCAG | SEQ ID NO: 51 |
| *putA-*1 | CAGGAAACAGCTATGACATGTTCTAGGGCATCGACGAA CCAG | SEQ ID NO: 52 |
| *putA-*2 | GAAATTGTTAAAAGCGCAGCGC | SEQ ID NO: 53 |
| *ABC-*F | GCTGCGCTTTTAACAATTTCGAAAACCCAGGATTGCTTT GTG | SEQ ID NO: 54 |
| *ABC-*R | TCGAAGCCGCACGTCATCTAG | SEQ ID NO: 55 |
| *putA-*3 | TAGATGACGTGCGGCTTCGATCCGTGAACGCCTATCTGT ACG | SEQ ID NO: 56 |
| *putA-*4 | TGTAAAACGACGGCCAGTGCGATCGATTCCACGCCCAA AC | SEQ ID NO: 57 |
| pK18-1 | GCACTGGCCGTCGTTTTAC | SEQ ID NO: 58 |
| pK18-2 | CATGTCATAGCTGTTTCCTGTGTG | SEQ ID NO: 59 |

### (2) Evaluation of proline productivity of strains modified by P_{pyc}-20 promoter mutant

To test the effect of applying the P*_{pyc}*-20 promoter mutant in *Corynebacterium glutamicum* on production of proline by strains, fermentation tests were performed in SLCgP1 and SLCgP2, respectively. The ingredients of the fermentation medium were as follows: glucose, 72 g/L; yeast powder, 1 g/L; soy peptone, 1 g/L; NaCl, 1 g/L; ammonium sulfate, 1 g/L; urea, 10 g/L; K₂HPO₄·3H₂O, 1 g/L; MgSO₄·7H₂O, 0.45 g/L; FeSO₄·7H₂O, 0.05 g/L; biotin, 0.4 mg/L; vitamin B1, 0.1 mg/L; MOPS, 40 g/L; and initial pH7.2. The strains were firstly inoculated into TSB liquid media and cultured for 8 h. The cultures were inoculated as seeds into a 24-well plate containing 800 µl of fermentation media in each well at an inoculation amount of 12 µl, and cultured at 30°C for 18 h. The rotating speed of the plate shaker was 800 rpm. Three samples were set for each strain. After the fermentation, the proline yields were measured. The results were listed in Table 7. The proline yields of the strains were significantly increased after the P*_{pyc}*-20 promoter expressing the expression cassette of *proB*^{G149K}, *proA* along with *proC* was inserted in the chromosome, and SLCgP2 was increased by 77% as compared to SLCgP1.

**Table 7**

| Strain | Proline Yield (g/L) |
|---|---|
| SLCgP1 | 3.10±0.15 |
| SLCgP2 | 5.48±0.23 |

The above results suggest that the mutants with enhanced *pyc* gene promoter may be used to express different target genes in *Corynebacterium glutamicum,* and applied to production of various products. Firstly, the mutants with enhanced *pyc* gene promoter may be used to enhance the expression of the *pyc* gene *per se* in *Corynebacterium glutamicum* to enhance the activity of Pyc, thereby reinforcing the synthesis from pyruvic acid to oxaloacetic acid, which may be applied to the production of target products dependent upon supply of oxaloacetic acid as the precursor, including biological production of amino acids with oxaloacetic acid as a major metabolic precursor, such as amino acids of the aspartic acid family (lysine, threonine, isoleucine, and methionine), amino acids of the glutamic acid family (glutamic acid, proline, hydroxyproline, arginine, and glutamine), and 5-aminolevulinic acid. The present disclosure has confirmed in the Examples that the mutants with enhanced *pyc* gene promoter can be used for production of lysine as an amino acid of the aspartic acid family and proline as an amino acid of the glutamic acid family. It has been verified that enhanced expression and activity of Pyc are useful for production of the above products, for example: 1) Peters-Wendisch PG *et al.* have reported that overexpression of Pyc could improve the yields of glutamic acid, lysine, and threonine ^{[5]}; 2) Pyc is a major enzyme in *Corynebacterium glutamicum* to catalyze the production of C4 oxaloacetic acid from C3 (PEP or pyruvic acid), and overexpression and enhanced activity of Pyc are important targets for production of amino acids with oxaloacetic acid as a precursor ^{[6]}; 3) the Patent reference^{[7]} has disclosed that enhanced activities of enzymes (phosphoenolpyruvate carboxylase or pyruvate carboxylase) that facilitate synthesis of oxaloacetic acid can improve the yield of 5-aminolevulinic acid. All *pyc* gene promoter mutants in the pyruvate carboxylase of *Corynebacterium glutamicum* in the present disclosure can be used to enhance the expression and activity of Pyc. Therefore, the *pyc* gene promoter mutants of the present disclosure may also be used for production of 5-aminolevulinic acid. Next, the Examples of the present disclosure also demonstrate that the mutants with enhanced *pyc* gene promoter may also be used for expression of genes such as *proB, proA,* and *proC,* indicating that the *pyc* gene promoter mutants of the present disclosure has good universality, which can be used for expression of more genes and thus production of more products.

### References:

[3] Wang, YC et al. Screening efficient constitutive promoters in Corynebacterium glutamicum based on time-series transcriptome analysis. Chinese Journal of Biotechnology, 2018, 34(11):1760 - 1771.
[4] CN112877269A
[5] Peters-Wendisch P G, Schiel B, Wendisch V F, et al. Pyruvate carboxylase is a major bottleneck for glutamate and lysine production by Corynebacterium glutamicum. [J]. J Mol Microbiol Biotechnol, 2001, 3(2):295 - 300.
[6] Uwe Sauer, Bernhard J. Eikmanns. The PEP-pyruvate-oxaloacetate node as the switch point for carbon flux distribution in bacteria [M]// FEMS Microbiology Reviews.
[7] CN103981203B

## Claims

1. A mutant of a pyruvate carboxylase gene promoter in *Corynebacterium glutamicum,* wherein the mutant is any one selected from the group consisting of the following (i) to (iv):
(i) the mutant has one or more mutated nucleotides in a core region corresponding to position 279 to position 317 of a promoter having a nucleotide sequence set forth in SEQ ID NO: 21;
(ii) comprising a reverse complementary sequence to the nucleotide sequence set forth in (i);
(iii) comprising a reverse complementary sequence to a sequence capable of hybridizing with the nucleotide sequence set forth in (i) or (ii) under high-stringent hybridization conditions or very high-stringent hybridization conditions; and
(iv) a nucleotide sequence having at least 90% sequence identity to the nucleotide sequence set forth in (i) or (ii),
wherein the nucleotide sequence of the mutant set forth in any one of (i) to (iv) is not CGATGTTTGATTGGGGGAATCGGGGGTTACGATACTAGG at positions corresponding to position 279 to position 317 of the sequence set forth in SEQ ID NO: 21; and preferably, the mutant set forth in any one of (i) to (iv) has an enhanced promoter activity as compared to a pyruvate carboxylase gene promoter having the sequence set forth in SEQ ID NO: 21.

2. The mutant of the pyruvate carboxylase gene promoter according to claim 1, wherein the nucleotide sequence of the mutant at positions corresponding to position 279 to position 317 of the nucleotide sequence set forth in SEQ ID NO: 21 is as follows:
NNNNNNTTGATTNNNNNNNNNNNNNNNTANNATNNNNNN, where N is selected from A, T, C, or G;
preferably, the mutant has an enhanced promoter activity of 1 to 17 folds or more as compared to the pyruvate carboxylase gene promoter having the sequence set forth in SEQ ID NO: 21.

3. The mutant of the pyruvate carboxylase gene promoter according to claim 1 or 2, wherein the nucleotide sequence of the core region of position 279 to position 317 of the mutant is one of the following sequences:
1) CTAATTTTGATTCGTACTGATTTCTGCTACGATGAGTCA;
2) GGATTGTTGATTTGAGCTTGATGAGCGTACAATCAACTT;
3) TTCTCCTTGATTGCGCCTTAACCGTGGTATGATTCGATA;
4) ATTGATTTGATTGGAACCTTACTGTGCTATGATTTGGTA;
5) TCGAGTTTGATTTCACAACGTGTGTGATAGGATATAATA;
6) TTGCGTTTGATTAAAGTATGCAAGGGCTAGTATGGTGAT;
7) ATCATTTTGATTCCGGCGCACATGTGGTAATATGGTATT;
8) TCGCCATTGATTGCCCGCCATCCATGCTATAATCGGAAG;
9) TTCCGCTTGATTGTGGCCATAGTATGATATTATTAATTA;
10) CGGATCTTGATTTTATGATGGGTATTGTATAATCTTGGT;
11) CGGATATTGATTTGGCCGGTGTTGTGGTAGTATCGTGTT;
12) AGGGGTTTGATTGGCCGCTCGGTGTGTTATCATGGAGAG;
13) GAGTTGTTGATTTCGTTGGTGCACGTATACAATGGTTTT;
14) CTTGGCTTGATTTTTGTTTGAGGGTTGTATAATGTTATT;
15) GACTAGTTGATTTCCGCCCTTGGTTGATATTATGCTTGA;
16) ATCCGCTTGATTTAGGCGTACGTTTAATAGTATATTGAA;
17) CGGGGCTTGATTTCCTTGTCGTGGCGTTATTATAATGGA;
18) ATGGAGTTGATTATACGATACTACAGATACTATACTGGT;
19) CCGTAGTTGATTGACTTGGGCAGTATATAGTATAATGAA; or
20) CGGGCCTTGATTGTAAGATAAGACATTTAGTATAATTAG.

4. The mutant of the pyruvate carboxylase gene promoter according to any one of claims 1 to 3, wherein the nucleotide sequence of the mutant is set forth in any one of SEQ ID NO: 1 to SEQ ID NO: 20.

5. An expression cassette, comprising the mutant of the pyruvate carboxylase gene promoter according to any one of claims 1 to 4.

6. An expression vector, comprising the mutant of the pyruvate carboxylase gene promoter according to any one of claims 1 to 4, or the expression cassette according to claim 5.

7. A recombinant host cell, comprising the mutant of the pyruvate carboxylase gene promoter according to any one of claims 1 to 4, the expression cassette according to claim 5, or the expression vector according to claim 6.

8. The recombinant host cell according to claim 7, the host cell is a genus *Enterobacter* or a genus *Corynebacterium,* preferably the genus *Corynebacterium,* further preferably *Corynebacterium glutamicum,* more specifically *Corynebacterium glutamicum* ATCC 13032, *Corynebacterium glutamicum* ATCC 13869, *Corynebacterium glutamicum* B253, *Corynebacterium glutamicum* ATCC 14067, and derived strains thereof.

9. Use of the mutant of the pyruvate carboxylase gene promoter according to any one of claims 1 to 4, the expression cassette according to claim 5, the expression vector according to claim 6, or the recombinant host cell according to claim 7 or 8 in at least one of the following (a) to (c):
(a) enhancing a transcriptional level of a gene, or preparing a reagent or kit for enhancing a transcriptional level of a gene;
(b) preparing a protein, or preparing a reagent or kit for use in the preparation of a protein; and
(c) producing a target product, or preparing a reagent or kit for use in the production of a target product;
optionally, the protein is a target product synthesis-associated protein, a membrane transport-associated protein, or a gene expression regulatory protein.

10. The use according to claim 9, wherein the target product is selected from at least one of amino acids and derivatives thereof; optionally, the amino acids and derivatives thereof are one or a combination of two or more selected from: proline, hydroxyproline, lysine, glutamic acid, arginine, ornithine, glutamine, threonine, glycine, alanine, valine, leucine, isoleucine, serine, cysteine, methionine, aspartic acid, asparagine, histidine, phenylalanine, tyrosine, tryptophan, 5-aminolevulinic acid, or derivatives of any one of the amino acids described above.

11. A method for enhancing expression of a target gene, wherein the method comprises operably ligating the mutant of the pyruvate carboxylase gene promoter according to any one of claims 1 to 4 to a target gene or a target RNA; optionally, the target RNA comprises at least one of tRNA or sRNA, and the target gene comprises at least one of a coding gene of a target compound synthesis-associated protein, a coding gene of a gene expression regulatory protein, or a coding gene of a membrane transport-associated protein;
optionally, the target gene comprises at least one of the following coding genes of enzymes: pyruvate carboxylase *pyc* gene, glutamate dehydrogenase *gdh* gene, aspartate kinase *lysC* gene, threonine operon *thrABC* gene, aspartate-semialdehyde dehydrogenase *asd* gene, aspartate-ammonia lyase *aspB* gene, homoserine dehydrogenase *hom* gene, homoserine O-acetyltransferase *metX* gene, dihydrodipicolinate synthase *dapA* gene, dihydrodipicolinate reductase *dapB* gene, meso-diaminopimelate dehydrogenase *ddh* gene, glutamate kinase *proB* gene, glutamate-5-semialdehyde dehydrogenase *proA* gene, pyrroline-5-carboxylate dehydrogenase *proC* gene, proline dehydrogenase/pyrroline-5-carboxylate dehydrogenase *putA* gene, glutamyl-t-RNAreductase *hemA* gene, phosphoenolpyruvate carboxylase *ppc* gene, amino acid transport protein *lysE* gene, ptsG system-associated coding gene, pyruvate dehydrogenase *aceE* gene, glyceraldehyde-3-phosphate dehydrogenase *gapN* gene, and lysine decarboxylase *cadA*/*ldcC* gene.

12. A method for preparing a protein, wherein the method comprises a step of expressing the protein using the expression cassette according to claim 5, the expression vector according to claim 6, or the recombinant host cell according to any one of claims 7 to 8; optionally, the protein is a target product synthesis-associated protein, a membrane transport-associated protein, or a gene expression regulatory protein; and
optionally, the method further comprises a step of isolating or purifying the protein.

13. A method for producing a target product, wherein the method comprises culturing a host cell containing the mutant of the pyruvate carboxylase gene promoter according to any one of claims 1 to 4 that is operably ligated to a target product synthesis-associated gene, and collecting the resulting target product; preferably the target product is an amino acid; preferably, the target product synthesis-associated gene is a gene associated with synthesis of the amino acid or a derivative thereof; and
optionally, the amino acid and derivative thereof are selected from one or a combination of two or more of: proline, hydroxyproline, lysine, glutamic acid, arginine, ornithine, glutamine, threonine, glycine, alanine, valine, leucine, isoleucine, serine, cysteine, methionine, aspartic acid, asparagine, histidine, phenylalanine, tyrosine, tryptophan, 5-aminolevulinic acid, or derivatives of any one of the amino acids described above.

14. A target compound according to claim 13, wherein the target compound uses oxaloacetic acid as a precursor; preferably, the target product using the oxaloacetic acid as the precursor comprises one or more of: lysine, threonine, isoleucine, methionine, glutamic acid, proline, hydroxyproline, arginine, glutamine, 5-aminolevulinic acid, pentanediamine, 5-aminovaleric acid, or a derivative of any one of the above.
